# EUROPEAN PATENT SPECIFICATION

(11) **EP 0 615 758 B1**
(45) Date of publication and mention of the grant of the patent: **22.09.1999**
(21) Application number: 93104490.3
(22) Date of filing: 18.03.1993
(51) Int. Cl.: A61K 39/00, C07K 17/10, C07K 2/00

(54) **Anti-tumor vaccines**
Antitumor Impfstoff
Vaccins antitumoraux

(43) Date of publication of application: 21.09.1994
(73) Proprietor: YEDA RESEARCH AND DEVELOPMENT CO. LTD., Rehovot 76100 (IL)
(72) Inventor: Shinitzky, Meir, Rehovot 76100 (IL)
(74) Representative: Grünecker, Kinkeldey, Stockmair & Schwanhäusser Anwaltssozietät

(56) References cited:
- EP-A- 0 261 648
- EP-A- 0 333 606
- WO-A-90/14353
- US-A- 4 931 275
- BIOLOGICAL ABSTRACT No.: 91132384 RAMAKRISHNA V. et al.:" Potentiation of delayed-type hypersensitivity response to syngeneic tumors in mice prevaccinated with cells modified by hydrostatic pressure and crosslinking."
- BIOLOGICAL ABSTRACT No.: 91132384 RAMAKRISHNA V. et al.:" Potentiation of delayed-type hypersensitivity response to syngeneic tumors in mice prevaccinated with cells modified by hydrostatic pressure and crosslinking."

## Description

### FIELD OF THE INVENTION

The present invention is generally in the field of cancer therapy and concerns novel immunogens,
a process for the preparation of said immunogens, an in vitro method of sensitizing immune cells, a vaccine preparation capable of inducing an anti-tumor immune response and the use of said vaccine preparation for the manufacture of a medicament.

The immunogens of the present invention comprise tumor cells which were treated to increase their specific immunogenicity and / or plasma membranes of such cells.

### BACKGROUND OF THE INVENTION AND PRIOR ART

As is well known and documented, cancer cells generally contain on their external surface specific neo-antigens which are foreign to the host body. Nevertheless, for reasons which are not entirely clear, the immune system fails to develop an effective immune reaction against the tumor cells. Attempts have been made to immunize cancer patients with preparations that will stimulate their immune systems to develop a reaction against the neo-antigens with the hope that such an immune reaction will destroy the residing cancer.

U.S. Patent No. 4,931,275 discloses anti-tumor vaccines which contain as active ingredient tumor cells which have been treated to augment their immunogenic properties, their plasma membranes or specific membrane proteins obtained from these cells or membranes. The treatment which augments their immunogenicity in accordance with this patent consists of either exposure of the cells to cholesteryl hemisuccinate which rigidifies the lipid layer of the plasma membrane, the application of hydrostatic pressure up to about 1500 atm, or a combination of these two treatments.

Ramakrishna & Shinitzky (*Cancer Immunol. Immunother.,* 1991, 33:1-8) showed that delayed type hypersensitivity (DTH) response to syngeneic tumors could be potentiated by cells treated with either hydrostatic pressure up to 1200 atm, crosslinking with adenosine 2',3'-dialdehyde, or with hydrostatic pressure followed by crosslinking.

It is an object of the present invention to provide a novel immunogen capable of inducing an anti-tumor immune response. More specifically, it is an object of the present invention, to provide such an immunogen which comprises modified tumor cells and/or plasma membranes thereof.

It is another object of the present invention to provide a process for preparing said immunogen.

It is a further object of the present invention to provide a vaccine comprising said immunogen.

### GENERAL DESCRIPTION OF THE INVENTION.

In accordance with the present invention it was found, that an immunogen having superior anti-tumor immunogenicity can be obtained from tumor cells treated by simultaneous exposure of a crosslinking agent being a 2',3'-nucleoside or nucleotide dialdehyde (hereinafter "said crosslinking agent"), and to hydrostatic pressure in the range of about 1200-1400 atm.

By one of its aspects the present invention provides an immunogen comprising modified tumor cells and capable of inducing an anti-tumor immune response, wherein said modified tumor cells have been prepared by exposing tumor cells simultaneously to said crosslinking agent at a concentration and for a time sufficient to cause crosslinking of proteins in the cell's plasma membranes and to hydrostatic pressure within the range of about 1200-1400 atm.

The concentration of said crosslinking agent is preferably above 20mM.

The application and release of pressure is preferably gradual, e.g. over a period of 5-10 minutes.

Said crosslinking agent is preferably a 2',3'-dialdehyde of a natural nucleotide or nucleoside as non-naturally occurring, i.e. synthetic nucleosides or nucleotides are very often highly toxic.

The preferred crosslinking agents are represented by the following formula I: 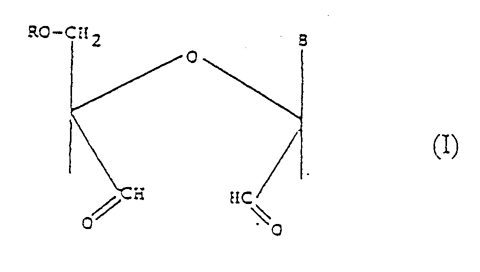 wherein
R is H, or a mono-, di-or tri-phosphate group, and
B is a nucleotide base selected from the group consisting of adenine, guanine, cytosine, thymine, uracil and inosine.

Examples of such a crosslinking agent are 2',3'-adenosine dialdehyde (AdA) and 2',3'-adenosine monophosphate dialdehyde (AMPdA).

The compound of formula I may be prepared by reacting a nucleoside or a nucleotide of the following formula II: 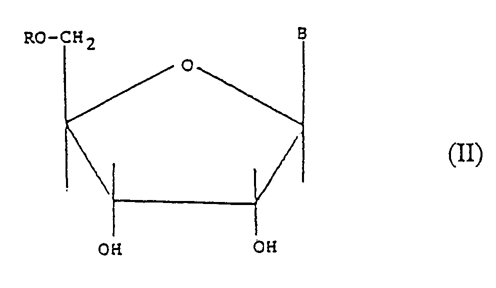 wherein R and B have the meanings given above for formula I, with an oxidizing agent, e.g. an alkali periodate.

The immunogen may consist of the whole modified tumor cells, or plasma membranes derived from, such cells, which substantially retain the capability of the modified tumor cells to induce the anti-tumor immune response.

Preferably, after the treatment in accordance with the invention, the modified tumor cells are exposed to high intensity radiation in order to destroy their genetic material. This is particularly important where the whole modified tumor cells are used for immunization and may not necessarily be required where said immunogen consists of membrane preparations.

By another aspect, the present invention provides a process for preparing an immunogen comprising modified tumor cells and/or membranes thereof and capable of inducing an anti-tumor immune response, said process comprising the steps of:
(a) providing tumor cells;
(b) exposing said tumor cells simultaneously to said crosslinking agent at a concentration and for a time sufficient to cause crosslinking of proteins in the cell's plasma membranes and to hydrostatic pressure within the range of about 1200-1400 atm; and
(c) separating between the tumor cells and said crosslinking agent.

Where the desired immunogen consists of the whole modified tumor cells, the product of the above process may be used *per se* or after several purification treatments, e.g. consisting of centrifugation and removal of the supernatant.

Where the desired immunogen consists of membranes of the modified tumor cells, the modified tumor cells are subjected to further treatment in which the cells are disrupted, e.g. by exposure to a hypotonic medium or by sonication, and then the membrane fragments are collected e.g. by centrifugation in a sucrose gradient, as generally known *per se*.

Said immunogen may be useful for the immunization of cancer patients against tumors, for the manufacture of a vaccine preparation or may be used for the sensitization of immune cells *in vitro*. For immunization, said immunogen may be injected into a patient together with a pharmaceutically acceptable carrier or adjuvant in an amount sufficient to achieve anti-cancer immune response.

For *in vitro* sensitization, immune cells, i.e. leukocytes or lymphocytes, are withdrawn from the patient by means known *per se* and then cultured together with said immunogen until a population of such immune cells reactive against said immunogen is obtained. Said population may then be reinjected into a cancer patient in order to treat his tumor.

By another of its aspects, the present invention thus provides a vaccine composition comprising said immunogen and a pharmaceutically acceptable carrier.

While the immunization of patients in accordance with the present invention can be performed by the use of an allogeneic immunogen, it is preferably performed by the use of an autologous immunogen, i.e. an immunogen comprising tumor cells of the same patient and/or membranes of said tumor cells: The use of an autologous immunogen entails significant advantages in that the immune response which occurs is primarily directed against the neo-antigen of the tumor, while where an allogenic immunogen is used the resulting immune response will be against all the non-self antigens of such an immunogen. A further advantage of the use of an autologous immunogen is in that the neo-antigens associated with a specific tumor may differ from one patient to another. Where an autologous preparation is used, the immunization of a patient comprises the following steps:
(a) withdrawing tumor growth from a patient by biopsy or surgery;
(b) dissociating intact tumor cells by mechanical or enzymatic means;
(c) dispersing said tumor cells in a medium;
(d) exposing said tumor cells simultaneously to said crosslinking agent at a concentration and for a time sufficient to cause crosslinking of proteins in the cell's plasma membranes and to hydrostatic pressure within the range of about 1200-1400 atm;
(e) removing the crosslinking agent and preparing a tumor-specific immunogen comprising the modified cells and/or membranes thereof and
(f) injecting said immunogen into the patient, whereby an anti-tumor immune response in said patient is induced.

Where an allogeneic immunogen is being used, an immunogen comprising modified tumor cells obtained from a defined tumor cell line may be used.

The invention will now be illustrated with reference to some non-limiting examples.

### BRIEF DESCRIPTION OF THE DRAWINGS

In the following, reference will at times be made to the accompanying drawings in which:

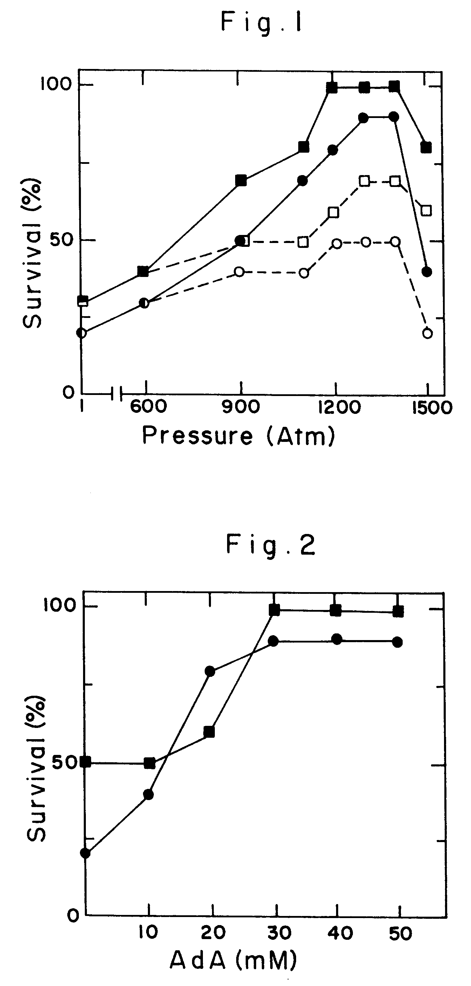

Fig. 1 is a graphic representation of an experiment in which the survivability of mice challenged with tumor cells after immunization with one of the following preparations was tested: EL4 leukemia cells treated with various levels of hydrostatic pressure in the presence of 40 mM AdA (filled squares); EL4 leukemia cells treated with increasing levels of hydrostatic pressure and then with 40 mM AdA (empty squares); B16 melanoma cells treated by various levels of hydrostatic pressure in the presence of 40 mM AdA (filled circles); and B16 melanoma cells treated with hydrostatic pressure and then with 40 mM AdA;
Fig. 2 shows the survivability of mice challenged with tumor cells following immunization with one of the following preparations: EL4 leukemia cells treated for 10 min with hydrostatic pressure of 1350 atm in the presence of various concentrations of AdA (filled squares); B16 melanoma cells treated in the same manner (filled circles);

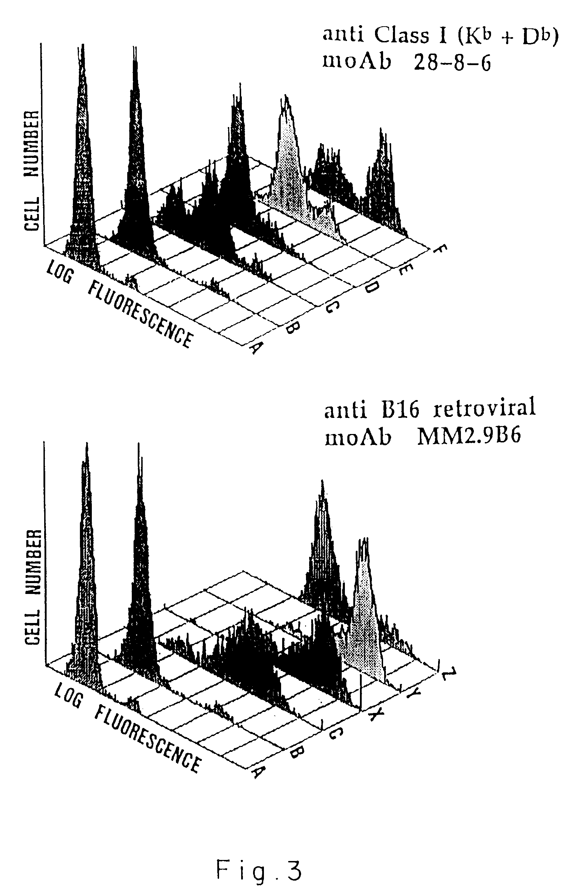

Fig. 3 shows a three-dimensional overlay of antigen expression on B16-BL6 melanoma cell plasma membrane surface as analyzed on FACScan by indirect immunofluorescence: A & B -negative controls (A - autofluorescence; B -cells reacted only with secondary antibody); C - unmodified cells; D & X -cells exposed to 20mM AdA; E & Z -cells exposed to hydrostatic pressure of 1,200 atm for 15 minutes; F & Y -cells exposed simultaneously to both 20 mM AdA and 1,200 atm pressure;

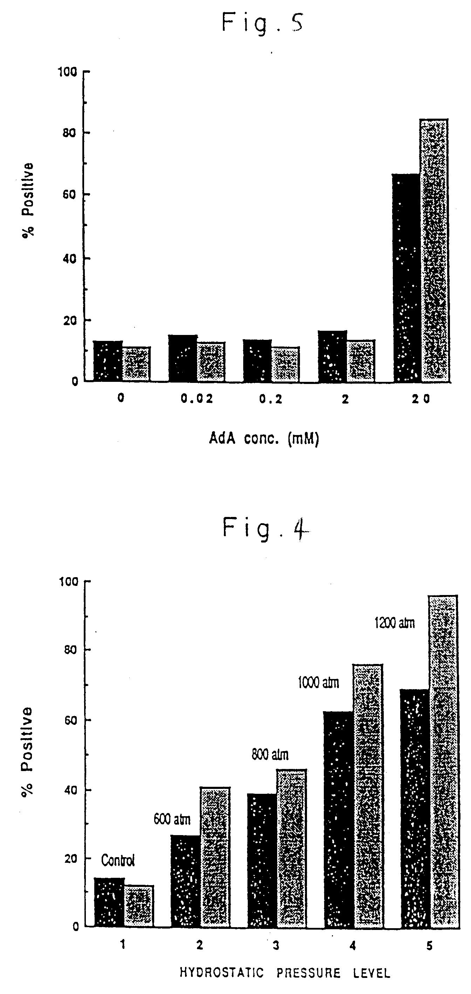

Fig. 4 shows the effect (as per cent of positive cells counted by FACScan instrument out of 10,000 events) of graded levels of hydrostatic pressure (applied for 15 minutes) combined with a constant dose of AdA 20 mM on antigen expression in B16-BL6 melanoma cells: filled bars - class I antigen, gray bars - B16 antigen; and
Fig. 5 shows the effect (per cent positive as determined on FACScan out of 10,000 events) of varying concentrations of AdA combined with a constant level of hydrostatic pressure (1200 atm, 15 minutes) on antigen expression in B16-Bl6 melanoma cells: black bars -MHC class I antigen, gray bars B16 antigen.

### EXAMPLE 1:

### Materials and Tumor Cells' Modification Methods

### Cells:

Cells from an EL4 tumor, which is a chemically induced T-leukemia (Gorrer, 1961. In: Harris RJG, ed., Biological Approach to Cancer Chemotherapy. Academic Press, New York) were maintained in ascites form in the peritoneal cavity of 6-8-week old C57B1/6J female mice. About 10⁵ cells were inoculated i.p. and 10 days later the cells (approximately 5x10⁸ cells per animal) were harvested.

Cells of ARadLV 136, which is a radiation-induced leukemogenic variant of ARadLV, were maintained *in vitro* as described previously (Haran-Ghera *et al.,* 1977, *J. Immunol.* **118**:600).

BL6 melanoma cells which are a very invasive variant of the B16 cell line (Hart 1979*, Am. J. Patholog.* **97**:587). The B16-BL6 tumor was serially passaged in syngeneic C57Bl mice by s.c. inoculation of 2 - 5 x 10⁶ cells.

### Adenosine 2',3'-dialdehyde (AdA):

AdA, which is a biologically compatible chemical crosslinker, was synthesized by a modification of the procedure previously described (Hansske *et al*., 1974, *Bioorg. Chem*. **3**:367): Adenosine (Sigma Chemical Co., St. Louis, Mo) and sodium metaperiodate (Fluka Chemie AG, Buchs. FRG) were mixed in 100 ml aqueous solution to a final concentration of 10 mM of each of these substances, stirred in the dark and cooled with ice water for 1 h, and then concentrated to 5 ml in vacuum at 30°C. The resulting concentrate was then incubated for 12 h at 4°C and the crystalline product which was obtained was separated and found to be homogeneous in thin-layer chromatography (silica gel G plate, 0.2 mm thickness, Merck Darmstadt; running solvent; acetonitrile/water, 4:1 v/v, R_{F} 0.80). The crystals were filtered, washed three times with cold water and dried over silica gel in vacuum (12 mm Hg; 1.6 kPa).

The yield of the above preparation procedure was found to be approximately 90%. The obtained product had a melting point of 110°C and melting was accompanied by decomposition, this being in agreement with previous reports (Hansske *et al*., 1974, *supra*).

### Modification of tumor cells:

After harvesting, the cells were washed twice with PBS (pH 7.4) and then subjected to one of the following modification treatments. The viability of the modified tumor cells was assessed by trypan blue dye exclusion.
***Modification I: crosslinking of proteins on tumor cell surface*** - About 10⁸ cells/ml were inoculated into a 50 ml tube (Falcon, Becton Dickinson Labware, N.J.) holding a PBS solution containing 0.5% AdA and were incubated in this solution for 1 h at room temperature with occasional mixing. Unbound AdA was removed by three cycles of centrifugation at 1500 rpm for 5 min, followed by gentle resuspending of the pellet in PBS (in the final resuspension PBS was added to obtain a desired cell concentration).
***Modification II: application of hydrostatic pressure*** - This modification was performed in a similar manner as previously described (Richert *et al*., 1986, *Cancer Immunol. Immunother*. **22**:119). Cells were dispersed in PBS at a concentration of 10⁸ cells/ml in a capped Eppendorf plastic tube (1.5 ml, Netheler and Hinz GmbH, Hamburg, FRG) and filled to the brim. A 0.5 in. (1.27 cm) 18G needle, was inserted through the cap and served as a vent for pressure equalization. Both the needle and the tube were filled with PBS and the cap was pressed down without entrapping any air bubbles (Removal of all air bubbles is important as these may cause cell disruption upon release of pressure). The tubes were then placed inside a pressure bomb of 40 ml capacity (Aminco, American Instruments Co., Md.), filled with PBS and sealed.
   Pressure was gradually applied to reach a level of about 1200 atm within 7-8 min and this pressure was maintained for about 15 min. Thereafter, the bomb was unlocked and allowed to decompress gradually to ambient pressure in about 8-10 min. The cells were then transferred into a 50 ml tube in 10 ml PBS and centrifuged at 1500 rpm for 5 min. The pellet was then gently resuspended in PBS to the desired concentration.
***Modification III: hydrostatic pressure and crosslinking in sequence*** - Cells were pressurized and then immediately crosslinked by the two modification procedures outlined above.
***Modification IV: hydrostatic pressure and crosslinking simultaneously*** - In this modification, which is the one performed in accordance with the invention cells were pressurized and crosslinked at the same time by the two modification treatments described above.

### EXAMPLE 2

The viability of C57B1 mice challenged with 10⁵ viable untreated tumor cells following pretreatment with various immunogenic preparations was tested. Survival after challenge was scored at day 30.

The pretreatment consisted of two vaccinations, one three weeks and the other one week prior to the challenge, with an immunogenic preparation which consisted of cells subjected to one of the following modification treatments: exposure to AdA, application of hydrostatic pressure or a combination of the two, by the modification procedures described above in Example 1. The cells used for vaccination were of the same kind as the cells used to challenge the mice.

The cells used in this experiment were either EL-4 of BL6.

### Test No. 1:

Four groups of mice were used, each pretreated with one of the following preparations:
- Group 1:: EL-4 leukemia cells treated for 10 minutes by various levels of hydrostatic pressure, in the presence of 40 mM AdA;
- Group 2:: EL-4 leukemia cells treated for 10 minutes by various levels of hydrostatic pressure and then by 40 mM AdA;
- Group 3:: B16 melanoma cells treated as Group 1;
- Group 4:: B16 melanoma cells treated as Group 2.

The results are shown in Fig. 1 (each point represents an average of 10 animals): Group 1 -filled squares; Group 2 -empty squares; Group 3 -filled circles; Group 4 -empty circles.

The results demonstrate that the maximal survivability was obtained after vaccination with an immunogenic preparation exposed to a hydrostatic pressure of 1200-1400 and surprisingly far inferior results were obtained after exposure to hydrostatic pressure of 1500 atm. Furthermore, these results show that the simultaneous exposure to both hydrostatic pressure and AdA leads to a higher survivability of the mice than these two treatments in sequence.

### Test No. 2:

Several groups of C57Bl mice (10 mice in each group) were pretreated by one of the following preparations:
- Treatment 1: EL-4 leukemia cells treated for 10 minutes by hydrostatic pressure of 1350 atm in the presence of various increasing concentrations of AdA;
- Treatment 2:: B16 melanoma cells treated as in Treatment 1.
- Treatment 3:: B16 melanoma cells treated for 10 min. by hydrostatic pressure of 1350 atm in the presence of various concentrations of AMPdA.

The results of the experiments relating to treatment 1 and treatment 2 are shown in Fig. 2 (each point represents an average of 10 animals): Treatment 1-filled squares; Treatment 2 -filled circles The results of treatments are not shown. In the experiments of Fig. 2, the maximal survival rate was obtained with an AdA or AMPdA concentration of above 30 mM.

### Test No. 3:

Five groups of C57B1 mice (6 mice in each group) were immunized subcutaneously and then challenged with B16-BL6 cells. The preparation used for immunization consisted of plasma membranes isolated by discontinuous sucrose gradient centrifugation (Maeda *et al*., 1983, *Biochim. Biosphys. Acta* 731:115) from untreated or treated cells. Details of the immunizing preparations is given in Table 1.

The following parameters were tested for each group of animals: survival rate; mean tumor diameter, measured using standard callipers in three orthogonal directions and presented by the mean value; metastatic nodules in lungs, scored after removal of the lungs, and fixations with Bouin's fixative and then washing with 70 ethanol. The results of this experiment are also shown in the following Table 1.

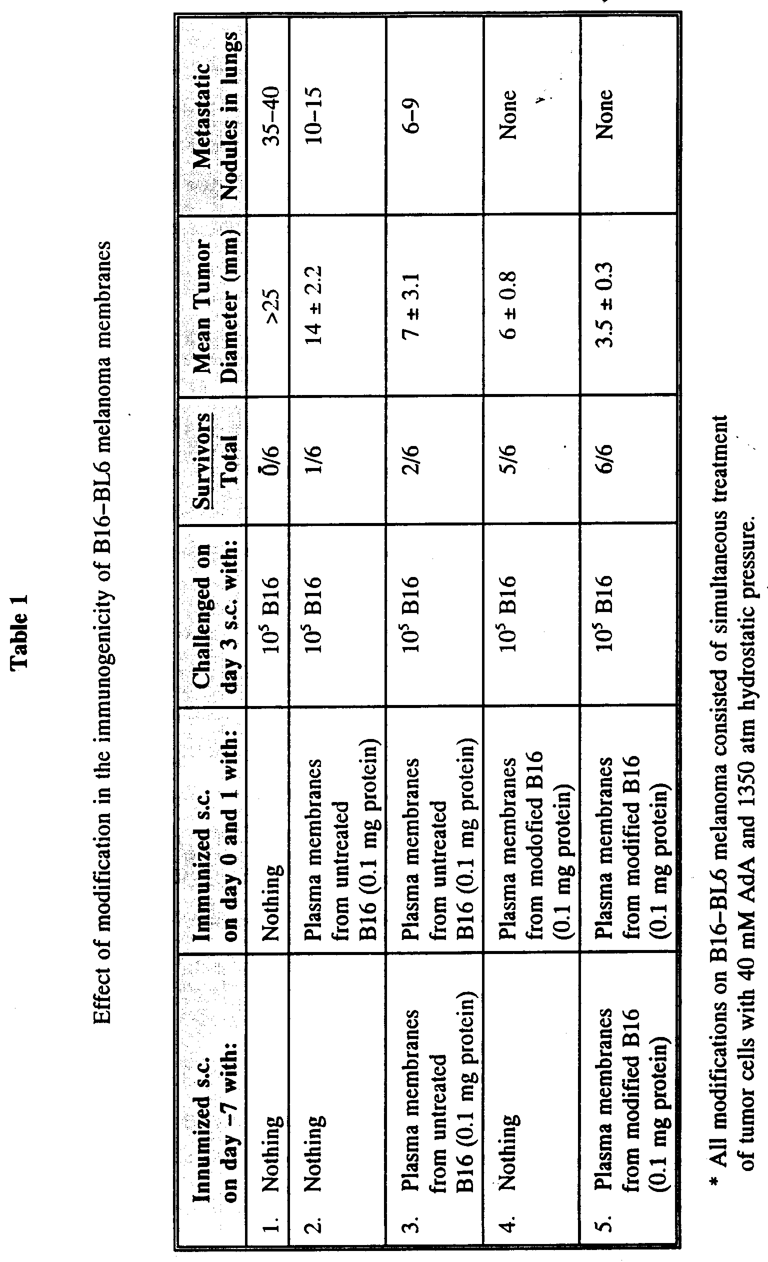

These results demonstrate that immunization with an immunizing preparation consisting of plasma membranes isolated from B16-BL6 cells treated by pressure and AdA in accordance with the invention (Treatments 4 and 5) brought a very high survival rate, the mean tumor diameter was minimal and no metastatic nodules in the lungs were observed.

These results prove the unexpected high potency of the vaccination treatment of the invention.

### EXAMPLE 3

The presence of MHC class I antigens (H-2k^{b}) and the tumor-specific retroviral antigen on B16-BL6 melanoma cells, derived from s.c. tumors either directly after obtaining single cell suspensions or passaging the cells in culture 6-8 times over a period of 3-4 weeks, was analyzed by flow cytometry.

About 10⁶ modified or unmodified B16-BL6 tumor cells were incubated at a first step with unlabelled primary antibodies: 30µl (25 µg) of anti-class I monoclonal antibody (clone 28-8-6, obtained from Dr. D. Sachs, National Cancer Institute, U.S.A.) or 10µl (8 µg) of monoclonal antibody against a retroviral antigen on the surface of B16 cells (MM2.9B6, Leong *et al.* 1988, *Cancer Res.* **48**:4954) in a final volume of 50 µl containing 1% FCS (fetal calf serum) and 0.01% of sodium azide and incubated for 45 minutes at 4°C. Cells were washed twice in HBS (hepes buffered saline) and the pellet was resuspended in a solution of a labeled secondary antibody: 50 µl of a solution containing 40 µg of the F(ab')2 fragment of FITC-GAMIG (fluorescein isothiocyanate labeled goat anti-mouse IgG) in HBS with 1% FCS and 0.1% sodium azide and incubated for an additional 45 minutes at 4°C in the dark. Thereafter cells were washed as before and fixed in 1% freshly prepared paraformaldelyde for 20 minutes. Fixed cells were washed thrice in HBS, resuspended in 0.5 ml HBS and passed through 100µ nylon mesh prior to flow cytrometric analysis. Samples were stored for no more than 24 hours in the dark at 4°C as samples stored for longer period gave rise to high autofluorescence.

Non-reacted B16-BL6 cells and B16-BL6 cells which reacted directly with FITC-GAMIG, and 2% BSA (bovine serum albumin) served as negative controls. Modified as well as unmodified cells were labeled and analyzed on either FACS 440 (Becton-Dickinson, Mountainview, CA) or on the FACScan instrument (Becton-Dickinson). Populations which were determined as positive on dual parameter (forward and orthogonal light scatter) analysis were gated and data was acquired on live gates. Histrograms were generated using either consort 40 software on FACS 440 or consort 30 with LYSYS software available with FACScan. Appropriate controls were introduced in order to apply logic threshold values. Amelanotic cells which appeared in the population were gated out in the present analysis by light scatter gating. Approximately 10,000 events were tested in each sample.

Exposure to AdA or to hydrostatic pressure was carried out in a similar manner to that described in Example 1.

As can be seen in Fig. 3, maximum expression of both class I and melanoma-specific antigen was noted with Bl6-BL6 cells which were modified by 1,200 atmospheres of hydrostatic pressure and simultaneous crosslinking with 20mM AdA.

In another set of experiments B16-BL6 cells were exposed to hydrostatic pressure in the range of 600-1,200 atm and to a constant concentration of AdA of 20mM and the results are shown in Fig. 4. In a further experiment B16-BL6 cells were exposed to a constant level of 1,200 atm of hydrostatic pressure while incubation with AdA at concentrations from 0.002mM to 20mM, and the results are shown in Fig. 5. As can be seen in these Figures, maximum fluorescent intensity for both class I and Bl6-BL6 tumor antigen was observed with the combination of a high level of pressure (1,200 atm) and the highest in the series of concentrations of AdA, 20mM.

### EXAMPLE 4

Immunogenicity of autologous tumor cells modified by a simultaneous pressure-crosslinking procedure was tested in five cancer patients, using the Delayed Type Hypersensitivity (DTH) test.

Tumor cells were isolated from each of five cancer patients and were subjected to a hydrostatic pressure of 1200 atm for 15 min. in the presence of 20 mM AdA in accordance with "Modification IV" in Example 1. The cells were then irradiated by a dose of 10,000 rads after which they lost their proliferation potential while remaining immunogenic.

10⁶ modified and irradiated autologous tumor cells (i.e. cells derived from patient's own tumor) were then injected intra dermally (I.D.). An immune reaction elicited by the injected cells was evidenced in the patients by a swollen red area which appeared in the skin at the inoculation site.

The degree of the patients' skin reaction was determined by the diameter of the erythema (redness) and induration (swelling) (according to the method of Skornick *et al., Cancer Immunol. Immunother*., **11****,** 93, 1981; a copy of this reference is attached hereto and marked "C"). The skin reaction was expressed by a score, "-", "+", "++" or "+++" where "-" indicated no reaction and "+++" indicated the maximal reaction. A standard maximal reaction was such obtained by injection with tuberculin (a lipoprotein of Mycobacterium tuberculosis) to people having a history of tuberculosis infection or previous vaccination.

The DTH reaction was monitored 24, 36, 48 and 72 hours after the inoculation of the cells. The peak of the reaction was normally observed 24-48 hours after inoculation.

The results were compared with control results obtained following injection by the same procedure of autologous irradiated but unmodified cells. These control cells were injected to the patient on the same days at an adjacent site.

The skin reaction was rated as described above by measuring the diameter of the erythema and the degree of induration at different periods of time after the injections and the results obtained with the five cancer patients is presented in the following Table 2:

**Table 2**

| **Patient** | **Skin reaction with non-modified irradiated cells** | | | | **Skin reaction with pressure & crosslinking modified irradiated cells** | | | |
|---|---|---|---|---|---|---|---|---|
| | **24h** | **36h** | **48h** | **72h** | **24h** | **36h** | **48h** | **72h** |
| 1. Melanoma stage I | ± | + | + | ± | ++ | +++ | ++ | + |
| 2. Melanoma stage IIa | ± | - | - | - | ++ | + | + | ± |
| 3. Melanoma stage III | ± | ± | - | - | ++ | ++ | +++ | + |
| 4. Colon carcinoma stage II | - | - | - | - | ++ | ++ | ++ | + |
| 5. Colon carcinoma stage II | - | ± | - | - | + | ++ | ++ | ± |

The results show that while non-treated cells cause a minimal DTH reaction (- or ±), the injection of the modified cells resulted in a very strong immune reaction in all patients (++ to +++). The maximal reaction appeared 36-48 hours after the injection of the cells in line with the expected progress of the DTH reaction.

## Claims

1. An immunogen comprising modified tumor cells, or plasma membranes obtained therefrom, and capable of inducing an anti-tumor immune response, wherein said modified tumor cells are prepared by exposing tumor cells simultaneously to thee action of a crosslinking agent, said crosslinking agent being a 2', 3'-nucleoside or nucleotide dialdehyde at a concentration and for a time sufficient to cause crosslinking of proteins in the cells' plasma membranes, and to hydrostatic pressure of about 1200 to 1400 atmospheres.

2. The immunogen according to claim 1, wherein said modified tumor cells are prepared by exposing tumor cells simultaneously to said hydrostatic pressure and to a crosslinking agent represented by the following formula (I): 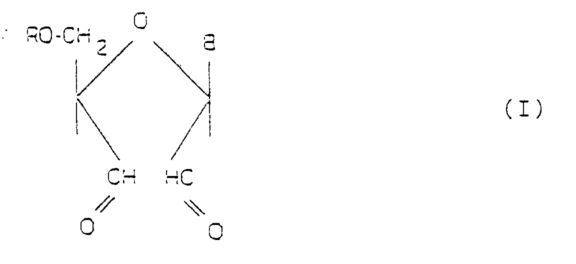 wherein R is H or a mono-, di-, or tri-phosphate group; and B is a nucleotide base selected from the group consisting of adenine, guanine, cytosine, thymine, uracil and inosine.

3. The immunogen according to claim 2, wherein said crosslinking agent is 2', 3'-adenosine dialdehyde or 2', 3'-adenosine monophosphate dialdehyde.

4. The immunogen according to any one of the preceding claims, wherein said crosslinking agent is at a concentration of 20 mM or above.

5. A process for preparing modified tumor cells according to claim 1 comprising the steps of:
(a) providing tumor cells;
(b) exposing tumor cells simultaneously to the action of a crosslinking agent, said crosslinking agent being a 2', 3'-nucleoside or nucleotide dialdehyde at a concentration and for a time sufficient to cause crosslinking of proteins in the cells' plasma membranes, and to hydrostatic pressure of about 1200 to 1400 atmospheres;
(c) separating the tumor cells and said crosslinking agent and, optionally, purifying the modified tumor cells thus obtained.

6. The process according to claim 5, wherein said crosslinking agent is at a concentration of 20 mM or above.

7. The process according to claim 5 or claim 6, wherein the modified cells are disrupted, followed by the collection of plasma membrane fragments.

8. An *in vitro* method of sensitizing immune cells to attack tumor cells, comprising withdrawing immune cells from a patient and culturing said immune cells with a tumor immunogen comprising modified tumor cells, or plasma membranes obtained therefrom, and capable of inducing an anti-tumor immune response, wherein said modified tumor cells are prepared by exposing tumor cells to a crosslinking agent being a 2', 3'-nucleoside or nucleotide dialdehyde at a concentration and for a time sufficient to cause crosslinking of proteins in the cells' plasma membranes, said culturing being for a time sufficient to obtain a culture of said immune cells reactive against said immunogen.

9. The method according to claim 8, wherein said 2', 3'-nucleoside or nucleotide dialdehyde crosslinking agent is represented by the following formula (I): 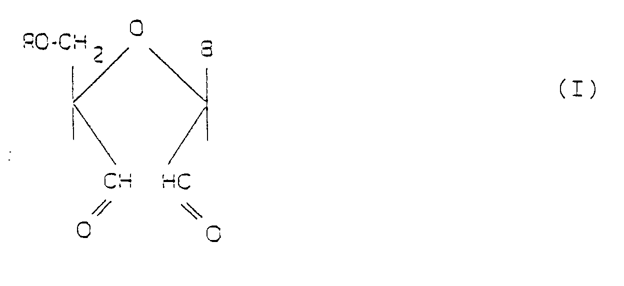 wherein R is H or a mono-, di-, or tri-phosphate group; and B is a base selected from the group consisting of adenine, guanine, cytosine, thymine, uracil and inosine.

10. The method according to claim 8, wherein said crosslinking agent is 2', 3'-adenosine dialdehyde or 2', 3'-adenosine monophosphate dialdehyde.

11. The method according to any of claims 8 to 10, wherein said crosslinking agent is at a concentration of 20 mM or above.

12. A vaccine preparation capable of inducing an anti tumor immune response comprising a pharmaceutically acceptable carrier and an effective amount of an immunogenic preparation comprising modified tumor cells, or plasma membranes obtained therefrom, wherein said modified tumor cells are prepared by exposing tumor cells simultaneously to the action of a crosslinking agent, said crosslinking agent being a 2', 3'-nucleoside or nucleotide dialdehyde at a concentration and for a time sufficient to cause crosslinking of proteins in the cells' plasma membranes, and to hydrostatic pressure of about 1200 to 1400 atmospheres.

13. Use of an immunogenic preparation comprising modified tumor cells, or plasma membranes obtained therefrom, wherein said modified tumor cells are prepared by exposing tumor cells simultaneously to the action of a crosslinking agent, said crosslinking agent being a 2', 3'-nucleoside or nucleotide dialdehyde at a concentration and for a time sufficient to cause crosslinking of proteins in the cells' plasma membranes, and to hydrostatic pressure of about 1200 to 1400 atmospheres for the manufacture of a vaccine preparation for the treatment of tumors.

## Patentansprüche

1. Immunogen, das modifizierte Tumorzellen oder daraus erhaltene Plasmamembranen umfaßt, und geeignet ist, eine Anti-Tumor-Immunantwort zu induzieren, wobei die modifizierten Tumorzellen dadurch hergestellt werden, daß Tumorzellen gleichzeitig der Wirkung eines Vernetzungsmittels, wobei das Vernetzungsmittel ein 2', 3'-Nucleosid- oder Nucleotiddialdehyd ist, in einer Konzentration und während eines Zeitraums, welche ausreichen, um die Vernetzung von Proteinen in den Plasmamembranen der Zellen zu bewirken, und einem hydrostatischen Druck von ungefähr 1200 bis 1400 Atmosphären ausgesetzt werden.

2. Immunogen nach Anspruch 1, wobei die modifizierten Tumorzellen dadurch hergestellt werden, daß Tumorzellen gleichzeitig dem hydrostatischen Druck und einem Vernetzungsmittel ausgesetzt werden, das durch die folgende Formel (I) dargestellt ist: 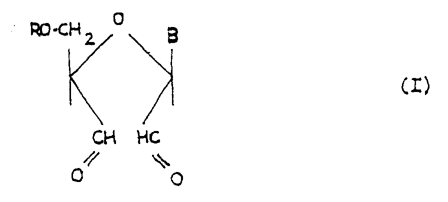 wobei R H oder eine Mono-, Di- oder Triphosphatgruppe ist; und B eine Nucleotidbase ist, die aus der Gruppe, bestehend aus Adenin, Guanin, Cytosin, Thymin, Uracil und Inosin, ausgewählt wird.

3. Immunogen nach Anspruch 2, wobei das Vernetzungsmittel 2', 3'- Adenosindialdehyd oder 2', 3'-Adenosinmonophosphatdialdehyd ist.

4. Immunogen nach einem der vorangehenden Ansprüche, wobei das Vernetzungsmittel in einer Konzentration von 20 mM oder höher vorliegt.

5. Verfahren zum Herstellen von modifizierten Tumorzellen nach Anspruch 1, umfassend die Schritte:
(a) Bereitstellen von Tumorzellen;
(b) gleichzeitiges Aussetzen der Tumorzeilen der Wirkung eines Vernetzungsmittels, wobei das Vernetzungsmittel ein 2', 3'-Nucleosid- oder Nucleotiddialdehyd ist, in einer Konzentration und für einen Zeitraum, welche ausreichen, um die Vernetzung von Proteinen in den Plasmamembranen der Zellen zu bewirken, und einem hydrostatischen Druck von ungefähr 1200 bis 1400 Atmosphären;
(c) Trennen der Tumorzellen und des Vernetzungsmittels und wahlweise Reinigen der so erhaltenen modifizierten Tumorzellen.

6. Verfahren nach Anspruch 5, wobei das Vernetzungsmittel in einer Konzentration von 20 mM oder höher vorliegt.

7. Verfahren nach Anspruch 5 oder Anspruch 6, in welchem die modifizierten Zellen aufgebrochen werden, gefolgt vom Sammeln der Plasmamembranfragmente.

8. *In vitro*-Verfahren zum Sensibilisieren von Immunzellen, um Tumorzellen anzugreifen, umfassend das Entnehmen von Immunzellen von einem Patienten und das Kultivieren dieser Immunzellen mit einem Tumorimmunogen, das modifizierte Tumorzellen oder daraus erhaltene Plasmamembranen umfaßt und geeignet ist, eine Anti-Tumor-Immunantwort zu induzieren, wobei die modifizierten Tumorzellen dadurch hergestellt werden, daß Tumorzellen einem Vernetzungsmittel, welches ein 2', 3'-Nucleosid- oder Nucleotiddialdehyd ist, in einer Konzentration und während eines Zeitraums ausgesetzt werden, welche ausreichen, um die Vernetzung von Proteinen in den Plasmamembranen der Zellen zu bewirken, wobei das Kultivieren während eines Zeitraums durchgeführt wird, der ausreicht, um eine Kultur der Immunzellen zu erhalten, die gegen das Immunogen reaktionsfähig sind.

9. Verfahren nach Anspruch 8, in welchem das 2', 3'-Nucleosid- oder Nucleotiddialdehyd-Vernetzungsmittel durch die folgende Formel (I) dargestellt ist: 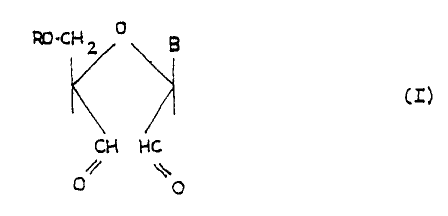 wobei R H oder eine Mono-, Di- oder Triphosphatgruppe ist; und B eine Base ist, die aus der Gruppe, bestehend aus Adenin, Guanin, Cytosin, Thymin, Uracil und Inosin, ausgewählt wird.

10. Verfahren nach Anspruch 8, wobei das Vernetzungsmittel 2', 3'-Adenosindialdehyd oder 2', 3'-Adenosinmonophosphatdialdehyd ist.

11. Verfahren nach einem der Ansprüche 8 bis 10, wobei das Vernetzungsmittel in einer Konzentration von 20 mM oder höher vorliegt.

12. Impfstoffzubereitung, die geeignet ist, eine Anti-Tumor-Immunantwort zu induzieren, umfassend einen pharmazeutisch annehmbaren Träger und eine wirksame Menge einer immunogenen Zubereitung, welche modifizierte Tumorzellen oder daraus erhaltene Plasmamembranen umfaßt, wobei die modifizierten Tumorzellen dadurch hergestellt werden, daß Tumorzellen gleichzeitig der Wirkung eines Vernetzungsmittels, wobei das Vernetzungsmittel ein 2', 3'-Nucleosid- oder Nucleotiddialdehyd ist, in einer Konzentration und während eines Zeitraums, welche ausreichen, um die Vernetzung von Proteinen in den Plasmamembranen der Zellen zu bewirken, und einem hydrostatischen Druck von ungefähr 1200 bis 1400 Atmosphären ausgesetzt werden.

13. Verwendung einer immunogenen Zubereitung, die modifizierte Tumorzellen oder daraus erhaltene Plasmamembranen umfaßt, wobei die modifizierten Tumorzellen dadurch hergestellt werden, daß Tumorzellen gleichzeitig der Wirkung eines Vernetzungsmittels, wobei das Vernetzungsmittel ein 2', 3'-Nucleosid- oder Nucleotiddialdehyd ist, in einer Konzentration und während eines Zeitraums, welche ausreichen, um die Vernetzung von Proteinen in den Plasmamembranen der Zellen zu bewirken, und einem hydrostatischen Druck von ungefähr 1200 bis 1400 Atmosphären ausgesetzt werden, für die Herstellung einer Impfstoffzubereitung für die Behandlung von Tumoren.

## Revendications

1. Immunogène comprenant des cellules tumorales modifiées ou des membranes plasmiques obtenues à partir de celles-ci, et capable d'induire une réponse immune antitumorale, dans lequel lesdites cellules tumorales modifiées sont préparées par exposition de cellules tumorales simultanément à l'action d'un agent réticulant, ledit agent réticulant étant un dialdéhyde de 2' ,3' -nucléoside ou nucléotide à une concentration et pendant un temps suffisants pour provoquer la réticulation de protéines dans les membranes plasmiques des cellules, et à une pression hydrostatique d'environ 1200 à 1400 atmosphères.

2. Immunogène selon la revendication 1, dans lequel lesdites cellules tumorales modifiées sont préparées par exposition de cellules tumorales simultanément à ladite pression hydrostatique et à un agent de réticulation représenté par la formule (I) suivante : 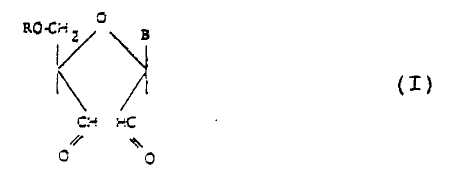 dans laquelle R est H ou un groupe mono-, di- ou triphosphate ; et B est une base nucléotidique choisie dans l'ensemble constitué par l'adénine, la guanine, la cytosine, la thymine, l'uracile et l'inosine

3. Immunogène selon la revendication 2, dans lequel ledit agent de réticulation est le dialdéhyde de 2' ,3'-adénosine ou le dialdéhyde de 2' ,3'-adénosine monophosphate.

4. Immunogène selon l'une quelconque des revendications précédentes, dans lequel ledit agent réticulant est à une concentration de 20 mM ou plus.

5. Procédé de préparation de cellules tumorales modifiées selon la revendication 1, comprenant les étapes de :
(a) mise à disposition de cellules tumorales ;
(b) exposition de cellules tumorales simultanément à l'action d'un agent de réticulation, ledit agent de réticulation étant un dialdéhyde de 2' ,3'-nucléoside ou nucléotide à une concentration et pendant un temps suffisants pour provoquer la réticulation de protéines dans les membranes plasmiques des cellules, et à une pression hydrostatique d'environ 1200 à 1400 atmosphères ;
(c) séparation des cellules tumorales et dudit agent de réticulation et, le cas échéant, purification des cellules tumorales modifiées ainsi obtenues.

6. Procédé selon la revendication 5, dans lequel ledit agent de réticulation est à une concentration de 20 mM ou plus .

7. Procédé selon la revendication 5 ou la revendication 6, dans lequel les cellules modifiées sont rompues, après quoi on recueille des fragments de membranes plasmiques.

8. Procédé de sensibilisation *in vitro* de cellules immunes pour attaquer des cellules tumorales, comprenant le prélèvement de cellules immunes sur un patient et la mise en culture desdites cellules inmunes avec un immunogène tumoral comprenant des cellules tumorales modifiées, ou des membranes plasmiques obtenues à partir de celles-ci, et capable d'induire une réponse immune antitumorale, dans lequel lesdites cellules tumorales modifiées sont préparées par exposition de cellules tumorales à un agent de réticulation qui est un dialdéhyde de 2' ,3' -nucléoside ou nucléotide à une concentration et pendant un temps suffisants pour provoquer la réticulation de protéines dans les membranes plasmiques des cellules, ladite mise en culture se faisant pendant un temps suffisant pour obtenir une culture desdites cellules immunes aptes à réagir vis-à-vis dudit immunogène.

9. Procédé selon la revendication 8, dans lequel ledit agent de réticulation dialdéhyde de 2' ,3' -nucléoside ou nucléotide est représenté par la formule (I) suivante : 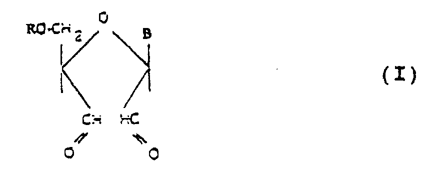 dans laquelle R est H ou un groupe mono-, di- ou triphosphate ; et B est une base choisie dans l'ensemble constitué par l'adénine, la guanine, la cytosine, la thymine, l'uracile et l'inosine.

10. Procédé selon la revendication 8, dans lequel ledit agent de réticulation est le dialdéhyde de 2' ,3' -adénosine ou le dialdéhyde de 2' ,3' -adénosine monophosphate.

11. Procédé selon l'une quelconque des revendications 8 à 10, dans lequel ledit agent de réticulation est à une concentration de 20 mM ou plus.

12. Préparation d'un vaccin capable d'induire une réponse immune antitumorale comprenant un véhicule pharmaceutiquement acceptable et une quantité efficace d'une préparation immunogène comprenant des cellules tumorales modifiées, ou des membranes plasmiques obtenues à partir de celles-ci, dans laquelle lesdites cellules tumorales modifiées sont préparées par exposition de cellules tumorales simultanément à l'action d'un agent de réticulation, ledit agent de réticulation étant un dialdéhyde de 2' ,3' - nucléoside ou nucléotide, à une concentration et pendant un temps suffisants pour provoquer la réticulation de protéines dans les membranes plasmiques de cellules, et à une pression hydrostatique d'environ 1200 à 1400 atmosphères.

13. Utilisation d'une préparation immnogène comprenant des cellules tumorales modifiées, ou des membranes plasmiques obtenues à partir de celles-ci, dans laquelle lesdites cellules tumorales modifiées sont préparées par exposition de cellules tumorales simultanément à l'action d'un agent de réticulation, ledit agent de réticulation étant un dialdéhyde de 2' ,3' -nucléoside ou nucléotide, à une concentration et pendant un temps suffisants pour provoquer la réticulation de protéines dans les membranes plasmiques de cellules, et à une pression hydrostatique d'environ 1200 à 1400 atmosphères, pour la fabrication d'une préparation de vaccin pour le traitement de tumeurs.
